Europäisches Patentamt

European Patent Office

Office européen des brevets

(11) Veröffentlichungsnummer: **0 464 274 B1**

(12) **EUROPÄISCHE PATENTSCHRIFT**

(45) Veröffentlichungstag der Patentschrift: **22.03.95**

(51) Int. Cl.6: **A61K 9/48**, A61K 35/78

(21) Anmeldenummer: **90250146.9**

(22) Anmeldetag: **06.06.90**

Die Akte enthält technische Angaben, die nach dem Eingang der Anmeldung eingereicht wurden und die nicht in dieser Patentschrift enthalten sind.

(54) **Weichgelatinekapsel.**

(43) Veröffentlichungstag der Anmeldung:
**08.01.92 Patentblatt 92/02**

(45) Bekanntmachung des Hinweises auf die Patenterteilung:
**22.03.95 Patentblatt 95/12**

(84) Benannte Vertragsstaaten:
**AT BE CH DE DK ES FR GB GR IT LI LU NL SE**

(56) Entgegenhaltungen:
EP-A- 0 265 338
EP-A- 0 343 575
DE-A- 2 614 864
FR-A- 2 626 471
US-A- 3 202 578

H.P. FIEDLER: "Lexikon der Hilfsstoffe für Pharmazie, Kosmetik und angrenzende Gebiete", 2. Auflage, Band 2, 1981, Editio Cantor, Aulendorf, DE

CHEMICAL ABSTRACTS, Band 101, Nr. 12, September 1984, Seite 407,Zusammenfassung Nr. 97593k, Columbus, Ohio, US; K.H. BAUER et al.: "Nonaqueous emulsions as vehicles for capsule fittings",& DRUG DEV. IND.PHARM. 1984, 10(5), 699-712

Klinisches Wörterbuch, W. Pschyrembel, W de G, Berlin-New York (1977), Seite 349

Römpp, Chemie Lexikon, 9. Auflage

(73) Patentinhaber: **Stephan, Günter**
**Happoldstrasse 47**
**D-70469 Stuttgart (DE)**

(72) Erfinder: **Stephan, Günter**
**Happoldstrasse 47**
**D-7000 Stuttgart 30 (DE)**
Erfinder: **Stephan, Dieter, Dr.**
**Gehrenwaldstrasse 35 A**
**D-7000 Stuttgart 60 (DE)**
Erfinder: **Nieder, Michael, Dr.**
**Gärtnerstrasse 13**
**D-7913 Senden (DE)**

(74) Vertreter: **Patentanwälte Wenzel & Kalkoff**
**Postfach 73 04 66,**
**Grubesallee 26**
**D-22124 Hamburg (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Weichgelatinekapsel mit einem in einem flüssigen Träger enthaltenen mittels Wasser und/oder Alkohol extrahierten Pflanzenextrakt.

Bei der Herstellung von Weichgelatinekapseln mit Pflanzenextrakten werden letztere in aller Regel in Form von Siccum-Extrakten, d.h. Trockenextrakten, eingesetzt, da Fluid-Extrakte wegen des Wasser- bzw. Alkoholgehalts nicht verwendet werden können. Um Trockenextrakte verkapseln zu können, muß der zu verkapselnde Inhalt in eine pumpbare Form gebracht werden. Dies geschieht dadurch, daß mit Hilfe von Pflanzenölen (Sojaöl, Rapsöl, Weizenkeimöl) und Emulgatoren (Lecithin und Bienenwachs) oder wie in der DE-PS 38 18 022.7-41 beschrieben mit Siliconöl eine stabile Emulsion hergestellt wird, die sich problemlos verkapseln läßt.

Bei der Auflösung einer solchen Kapsel im Magen oder Darm, je nach Behandlung der Gelatine-Hülle, spreidet die Emulsion zwar gut auf der Magen- oder Darmschleimhaut, jedoch ist die Aufnahme des Extrakts, der ja erst aus der öligen Emulsion herausgelöst werden muß stark verzögert.

Es ist bekannt, Pflanzenextrakte mit Öl zu extrahieren (EP-A3-0 265 338). Dabei handelt es sich um aus der Sägepalmenfrucht gepreßte Extrakte. Da ein solches Öl, wie in jener Druckschrift beschrieben, üblicherweise hydrophob ist, wird ein Zusatz von Polyethylenglykol (im folgenden kurz PEG) verwendet, um solche Extrakte hydrophil zu machen, wodurch eine bessere Resorbierbarkeit des verkapselten, allein durch Pressen gewonnenen Pflanzenextraktes erzielt werden soll.

Bei chemischen Reinsubstanzen, insbesondere Digoxin, ist es bekannt (DE-OS 26 14 864), Glycerin als Stabilisator für eine Lösung solcher Substanzen einzusetzen. Damit soll für solche speziellen Substanzen eine Lösung zur Verfügung gestellt werden, durch die sich im Vergleich zu Tabletten, die den Ausgangspunkt jener Entwicklung darstellen, eine bessere Resorbierbarkeit ergeben.

Die GB-PS 1 135 709 beschreibt die Verbesserung der Aktivitätsstabilität von Enzymen, nämlich Bromelain. Um solche Enzyme in quasi flüssigem, gelöstem Zustand zu halten und eine irreversible Aktivitätsminderung infolge Auskristallisieren zu verhindern, können verschiedene Substanzen, wie beispielsweise physiologisch verträgliche Lipoide und/ oder Paraffin, die jeweils bei Umgebungstemperatur flüssig sein sollen, und/oder PEG verwendet werden. Es geht hierbei allein um Stabilisierungsprobleme von Stoffen, die eine völlig andere Beschaffenheit und Wirkung als Pflanzenextrakte haben, so daß für deren Verarbeitung bzw. Aufbereitung dieser Druckschrift keine Anregung zu entnehmen ist.

Um für Grubenotter-Gifte spezielle orale Verabreichungsformen bereitzustellen, ist u.a. PEG als Suspensionsmittel bekannt (EP-A1-0 243 716). Dieses ist unter anderem als die Homogenität des Kapselinhaltes verbessernder Suspensionsvermittler beschrieben.

Bei einem unter Verwendung von Pflanzenextrakten hergestellten Laxans ist als Suspensionsmittel ein Polymer des Typs Pluronics (Polyoxyethylenpolyoxypropylen) bekannt geworden (US-PS 3 202 578).

Bei Weichgelatinekapseln, die als Wirkstoff Giftstoffe in Form von Mutterkornalkaloiden enthalten, d.h. ein klar definiertes Wirkstoffgemisch aus dem Mutterkornpilz, ist es bekannt (CH-PS 655 848), zur Stabilisierung solcher Ergotalkaloide, die sonst für sich in der Kapsel wegen der chemischen Instabilität des Gemisches nicht beständig wären, PEG einzusetzen. Diese Instabilität wird durch Isomerisierungen am Ergotamin-Gerüst verursacht, die in Lösungen selbständig stattfinden. PEG-Lösungen von Ergotalkaloiden besitzen eine stark herabgesetzte Isomerisierungstendenz, so daß sie zur Herstellung von Arzneiformen mit befriedigender Haltbarkeit eingesetzt werden können. Mit anderen Worten dient das Versetzen solcher Gemische mit Propylenglykol und PEG der Herstellung vernünftiger Verabreichungsformen ohne Blick auf die pharmakologische Wirkung.

Ausgangspunkt der vorliegenden Erfindung ist demgegenüber eine Weichgelatinekapsel mit einem mittels Wasser und/oder Alkohol extrahierten Pflanzenextrakt, und es liegt der Erfindung die Aufgabe zugrunde, derartige Pflanzenextrakt-Weichgelatinekapseln hinsichtlich ihrer Bioverfügbarkeit in kostengünstiger, einfacher Weise zu verbessern, indem eine Optimierung zwischen der Substanz und ihrem Träger geschaffen wird.

Diese Aufgabe wird erfindungsgemäß dadurch gelöst, daß der flüssige Träger des Pflanzenextrakts ein Lösungsmittel für diesen aus der Gruppe der polykondensierten Alkohole, Polyole oder deren Monomethyl- bzw. Monoethylether ist, wobei vorteilhaft dem Träger-Lösungsmittel biologische komplexbildende Lösungsvermittler der Phosphatidgruppe beigefügt sein können. Im Gegensatz zu Wasser, das als Lösungsmittel in Weichgelatinekapseln aus Verträglichkeitsgründen (Lösen der Gelatine) nicht eingesetzt werden kann, verbinden die erfindungsgemäß vorgeschlagenen Lösungsmittel in überraschender Weise bei den sehr heterogenen Pflanzenextrakten die Eigenschaften, sowohl als besonders geeignete Lösungsmittel, die mit der Gelatine kompatibel sind und für die hydrophil extrahierten Pflanzenextrakte eine dem Wasser vergleichbare Lösungsfähigkeit aufweisen, eingesetzt werden zu können als auch die Resorptionsfähigkeit in einem Maße zu steigern, wie dies bisher nur von im wesentlichen homogenen chemischen Stoffen oder Natursubstanzen her bekannt ist, ohne daß beispielsweise andere wesentliche Eigenschaften wie die Mischbarkeit gemindert werden. Zudem besitzen diese Lösungsmittel noch positive Stabilisierungseigenschaften für die Pflanzenextrakte.

Als besonders bevorzugte Ausführungsmöglichkeiten für polykondensierten Alkohol haben sich PEG sowie Polyglycerin erwiesen.

Zwar ist PEG ganz generell als Mittel zur Verbesserung der Bioverfügbarkeit gegenüber Emulsionen auf Öl-Wachs-Basis bekannt (K. Bauer et al. "Drug Dev. Ind. Pharm. 1984, 10(5), 699-712" oder Chemical Abstracts, Band 101, Nr. 12, September 1984, Zusammenfassung Nr. 97593k, Columbus, Ohio, US; "Nichtwässrige Emulsionen als Vehikel für Kapselfüllungen"), ohne daß dabei die spezifischen Probleme heterogener Pflanzenextrakte erkannt und einer Lösung zugeführt wären.

Vielmehr wird mit Hilfe der erfindungsgemäßen Erkenntnis nicht nur eine bessere Bioverfügbarkeit geschaffen, sondern es kann bei Lösung der benötigten Extraktmenge insbesondere in PEG ganz auf Emulgatoren verzichtet werden, und selbst dann, wenn nicht genügend Pflanzenextrakt direkt in dem polykondensierten Alkohol gelöst wird, kann die Kapselgröße wesentlich verringert werden, da sich ein verbessertes Verhältnis zwischen dem Pflanzenextrakt und den Hilfsstoffen (Pflanzenölen) ergibt. Bezüglich der Lösung in Polyglycerin ist hervorzuheben, daß rein alkoholisch ausgezogene Extrakte sich darin lösen, wobei sich allerdings solche Lösungen bzw. Emulsionen nicht direkt verkapseln lassen, weil das Glycerin durch die Gelatineöle diffundiert und als Folge davon die Kapsel einfallen kann. Eine solche Mischung wird mit PEG versetzt, wobei sich in Glycerin emulgierte Extrakte in jedem Verhältnis mit PEG mischen lassen und die Vorteile des PEG dann auch eintreten, also insbesondere die Resorption der Lösung gegenüber der reinen Emulsion schon im Magentrakt und damit schneller erfolgen kann.

Als Ausführungsbeispiele der Erfindung wurden im Laborversuch folgende verkapselungsfähige Suspensionen für Pflanzenextrakte erzeugt:

1) Aus Extrakt Agnus Castus (85.683 der Fa. Flachsmann), Auszugsmittel 50 Vol % Ethanol, und aus Polyethylenglykol 400 wird eine 70 %ige Suspension hergestellt.

Die Suspension wird auf dem Laborwalzenstuhl homogenisiert und im Exsikkator entlüftet. Es ergibt sich eine stabile, homogene, gut zu verkapselnde Suspension.

2) Aus Extrakt Crataegi sicc (85.285 Fa. Flachsmann), Auszugsmittel 70 Vol % Ethanol, und aus Polyethylenglykol 400 wird eine 60 %ige Suspension hergestellt.

Die weitere Verarbeitung erfolgt wie im Beispiel 1).

3) Aus Extrakt Harpagophytum sicc (85.566 Fa. Flachsmann), Auszugsmittel 30 Vol % Ethanol, und aus Polyethylenglykol 400 wird eine 70 %ige Suspension hergestellt.

Die weitere Verarbeitung erfolgt wie im Beispiel 1).

4) Aus Extrakt Salicis 20 % Salicin (Charge 1725 Paninkret), Auszugsmittel 20 Vol % Ethanol, und aus Polyethylenglykol 400 wird eine 70 %ige Mischung hergestellt.

Die weitere Verarbeitung erfolgt wie im Beispiel 1).

5) Aus Extrakt Liquiritia (85.810 Fa. Flachsmann Charge 9E 543), Auszugsmittel Wasser, und aus Polyethylenglykol 400 wird eine 80 %ige Mischung hergestellt.

Die weitere Verarbeitung erfolgt wie im Beispiel 1).

6) Im folgenden sind tabellarische Zusammenstellungen wiedergegeben, die die Ergebnisse der Lösungsfähigkeit erfindungsgemäß zusammengesetzter verkapselbarer Stoffe zeigen, die, wie im Beispiel 1) angegeben, hergestellt wurden und jeweils eine Gruppe von zehn (6.1 - 6.3) bzw. zwölf (6.4 - 6.9) Pflanzenextrakten in neun verschiedenen Lösungsmitteln umfassen:

6.1 Lösungsmittel Polyethylenglykol 400

```
Pflanzentrockenext.
Bezeichnung     Menge   Lösungsm.  Prozentanteil  Bemerkung
                in g    in g       d.Pflanzenext.
```

| Pflanzentrockenext. Bezeichnung | Menge in g | Lösungsm. in g | Prozentanteil d.Pflanzenext. | Bemerkung |
|---|---|---|---|---|
| Agnus Castus | 70 | 30 | 70 | gut verkapselbar |
| Crataegus | 60 | 40 | 60 | " |
| Harpagophytum | 70 | 30 | 70 | " |
| Liquiritiae | 80 | 20 | 80 | " |
| Salicis | 70 | 30 | 70 | " |
| Echinacea | 70 | 30 | 70 | " |
| Bulbus allii | 70 | 30 | 70 | " |
| Sabal serrulata | 80 | 20 | 80 | " |
| Ammi Visnagae | 60 | 40 | 60 | " |
| Fumaria | 70 | 30 | 70 | " |

6.2 Lösungsmittel Diethylenglykol-Monoethylether

| Pflanzentrockenext. Bezeichnung | Menge in g | Lösungsm. in g | Prozentanteil d.Pflanzenext. | Bemerkung |
|---|---|---|---|---|
| Agnus Castus | 80 | 20 | 80 | gut verkapselbar |
| Crataegus | 70 | 30 | 70 | " |
| Harpagophytum | 70 | 30 | 70 | " |
| Liquiritiae | 60 | 40 | 60 | " |
| Salicis | 80 | 20 | 80 | " |
| Echinacea | 60 | 40 | 60 | " |
| Bulbus allii | 70 | 30 | 70 | " |
| Sabal serrulata | 60 | 40 | 60 | " |
| Ammi Visnagae | 60 | 40 | 60 | " |
| Fumaria | 60 | 40 | 60 | " |

6.3 Lösungsmittel Diethylenglykol-Monoethylether mit 2 Gew.-% Lecithin

| Pflanzentrockenext. | | | | |
|---|---|---|---|---|
| Bezeichnung | Menge in g | Lösungsm. in g | Prozentanteil d.Pflanzenext. | Bemerkung |
| Agnus Castus | 80 | 20 | 80 | gut verkapselbar |
| Crataegus | 70 | 30 | 70 | " |
| Harpagophytum | 70 | 30 | 70 | " |
| Liquiritiae | 60 | 40 | 60 | " |
| Salicis | 80 | 20 | 80 | " |
| Echinacea | 80 | 20 | 80 | " |
| Bulbus allii | 80 | 20 | 80 | " |
| Sabal serrulata | 80 | 20 | 80 | " |
| Ammi Visnagae | 70 | 30 | 70 | " |
| Fumaria | 70 | 30 | 70 | " |

6.4 Lösungsmittel Polyglycerin

| Pflanzentrockenext. | | | | |
|---|---|---|---|---|
| Bezeichnung | Menge in g | Lösungsm. in g | Prozentanteil d.Pflanzenext. | Bemerkung |
| Agnus Castus | 60 | 40 | 60 | gut verkapselbar |
| Crataegus | 70 | 30 | 70 | " |
| Harpagophytum | 70 | 30 | 70 | " |
| Liquiritiae | 70 | 30 | 70 | " |
| Salicis | 80 | 20 | 80 | " |
| Echinacea | 80 | 20 | 80 | " |
| Bulbus allii | 60 | 40 | 60 | " |
| Sabal serrulata | 60 | 40 | 60 | " |
| Ammi Visnagae | 60 | 40 | 60 | " |
| Fumaria | 70 | 30 | 70 | " |
| Gelee royal | 80 | 20 | 80 | " |
| Hippocastanii | 70 | 30 | 70 | " |

6.5 Lösungsmittel Polyglycerin + 5 % Sorbit

| Pflanzentrockenextr. | | | | |
|---|---|---|---|---|
| Bezeichnung | Menge in g | Lösungsm. in g | Prozentanteil d.Pflanzenext. | Bemerkung |
| Agnus Castus | 60 | 40 | 60 | gut verkapselbar |
| Crataegus | 80 | 20 | 80 | " |
| Harpagophytum | 70 | 30 | 70 | " |
| Liquiritiae | 80 | 20 | 80 | " |
| Salicis | 80 | 20 | 80 | " |
| Echinacea | 80 | 20 | 80 | " |
| Bulbus allii | 60 | 40 | 60 | " |
| Sabal serrulata | 60 | 40 | 60 | " |
| Ammi Visnagae | 70 | 30 | 70 | " |
| Fumaria | 70 | 30 | 70 | " |
| Gelee royal | 80 | 20 | 80 | " |
| Hippocastanii | 70 | 30 | 70 | " |

6.6 Lösungsmittel Diglycerinmonomethylether

| Pflanzentrockenext. | | | | |
|---|---|---|---|---|
| Bezeichnung | Menge in g | Lösungsm. in g | Prozentanteil d.Pflanzenext. | Bemerkung |
| Agnus Castus | 60 | 40 | 60 | gut verkapselbar |
| Crataegus | 60 | 40 | 60 | " |
| Harpagophytum | 70 | 30 | 70 | " |
| Liquiritiae | 60 | 40 | 60 | " |
| Salicis | 60 | 40 | 60 | " |
| Echinacea | 60 | 40 | 60 | " |
| Bulbus allii | 70 | 30 | 70 | " |
| Sabal serrulata | 80 | 20 | 80 | " |
| Ammi Visnagae | 60 | 40 | 60 | " |
| Fumaria | 60 | 40 | 60 | " |
| Gelee royal | 60 | 40 | 60 | " |
| Hippocastanii | 60 | 40 | 60 | " |

6.7 Lösungsmittel Diglycerinmonoethylether

| Pflanzentrockenext. | | | | |
|---|---|---|---|---|
| Bezeichnung | Menge in g | Lösungsm. in g | Prozentanteil d.Pflanzenext. | Bemerkung |
| Agnus Castus | 70 | 30 | 70 | gut verkapselbar |
| Crataegus | 60 | 40 | 60 | " |
| Harpagophytum | 70 | 30 | 70 | " |
| Liquiritiae | 60 | 40 | 60 | " |
| Salicis | 60 | 40 | 60 | " |
| Echinacea | 60 | 40 | 60 | " |
| Bulbus allii | 70 | 30 | 70 | " |
| Sabal serrulata | 80 | 20 | 80 | " |
| Ammi Visnagae | 60 | 40 | 60 | " |
| Fumaria | 70 | 30 | 70 | " |
| Gelee royal | 60 | 40 | 60 | " |
| Hippocastanii | 60 | 40 | 60 | " |

6.8 Lösungsmittel Tetraglycerin + 5 % Cephalin

| Pflanzentrockenext. | | | | |
|---|---|---|---|---|
| Bezeichnung | Menge in g | Lösungsm. in g | Prozentanteil d.Pflanzenext. | Bemerkung |
| Agnus Castus | 70 | 30 | 70 | gut verkapselbar |
| Crataegus | 70 | 30 | 70 | " |
| Harpagophytum | 70 | 30 | 70 | " |
| Liquiritiae | 60 | 40 | 60 | " |
| Salicis | 80 | 20 | 80 | " |
| Echinacea | 60 | 40 | 60 | " |
| Bulbus allii | 80 | 20 | 80 | " |
| Sabal serrulata | 80 | 20 | 80 | " |
| Ammi Visnagae | 70 | 30 | 70 | " |
| Fumaria | 70 | 30 | 70 | " |
| Gelee royal | 60 | 40 | 60 | " |
| Hippocastanii | 80 | 20 | 80 | " |

6.9 Lösungsmittel PEG 400 + 5 % Cephalin + 5 % Isosorbit

| Pflanzentrockenext. | | | | |
|---|---|---|---|---|
| Bezeichnung | Menge in g | Lösungsm. in g | Prozentanteil d.Pflanzenext. | Bemerkung |
| Agnus Castus | 80 | 20 | 80 | gut verkapselbar |
| Crataegus | 70 | 30 | 70 | " |
| Harpagophytum | 70 | 30 | 70 | " |
| Liquiritiae | 80 | 20 | 80 | " |
| Salicis | 80 | 20 | 80 | " |
| Echinacea | 70 | 30 | 70 | " |
| Bulbus allii | 70 | 30 | 70 | " |
| Sabal serrulata | 80 | 20 | 80 | " |
| Ammi Visnagae | 70 | 30 | 70 | " |
| Fumaria | 80 | 20 | 80 | " |
| Gelee royal | 70 | 30 | 70 | " |
| Hippocastanii | 80 | 20 | 80 | " |

Zusammenfassend läßt sich feststellen, daß sich jeweils mindestens 60 %ige Mischungen alkoholisch oder wässrig ausgezogener Pflanzenextrakte herstellen lassen, die stabil und gut zu verkapseln sind. Dabei sind folgende Vorteile gegenüber bekannten Suspensionen maßgeblich:

1. Die Suspensionen wurden ohne Zusatz von Emulgatoren hergestellt.

2. Die Pflanzenextrakte liegen jeweils zumindest in Teilen gelöst vor, d.h. sie sind mit Wasser in beliebigen Verhältnis weitermischbar, so daß eine gute Bioverfügbarkeit als gesichert angenommen werden kann.

3. Solche hohen Wirkstoffanteile konnten mit den bisher verwendeten Öl/Lecithingemischen nicht erreicht werden. Bei diesen galt bisher die Faustregel, daß max. 50 % des Füllgewichtes pulvrige Substanzen sein können; der Rest sind ölige Hilfsstoffe oder ölige Wirkstoffe.

Um dies zu untermauern, wurden einige Analysenzertifikate von den Firmen Swiss Caps und Pharmagel für Suspensionskapseln ausgewertet:

a) Hersteller SCA

| Name | Wirkstoffe | mg/Kapsel | gesamt | % |
|------|-----------|-----------|--------|---|
| Prostamine | Extrakte | 542,50 | 980,00 | 55,4 |
| Regarol | Extrakte | 196,00 | 1070,00 | 18,3 |
| Liverbron | Extr./Vitamine | 271,73 | 948,39 | 28,7 |
| Beruhig. | Extrakte | 130,00 | 290,00 | 44,8 |
| Teufelskr. | Droge | 200,00 | 440,00 | 45,5 |
| Propolis | Extrakt | 100,00 | 245,00 | 40,8 |
| Vit./Hormon | Vitamin/Mineralien | 107,76 | 415,00 | 26,0 |
| Magnesium | Mineralien | 300,00 | 829,50 | 36,2 |
| Schlankh. | Extrakte | 130,00 | 290,00 | 44,8 |

b) Hersteller Pharmagel

| Name | Wirkstoffe | mg/Kapsel | gesamt | % |
|------|-----------|-----------|--------|---|
| Erytoxinal | Vitamine/Antibiotikum | 266,04 | 600,00 | 44,3 |
| Chelidonii | Extrakt | 100,00 | 400,00 | 25,0 |
| Fumaria | Extrakt | 200,00 | 422,00 | 47,4 |
| Prostalex | Extrakt | 542,50 | 1120,00 | 48,4 |
| G.P.E. | Extrakt/Vitamine | 320,00 | 682,00 | 46,9 |
| Ginseng | Droge | 100,00 | 350,00 | 28,6 |
| Aviton | Vitamine/Mineralien | 417,55 | 1050,00 | 39,8 |
| Pollen | Extrakt | 156,66 | 910,00 | 17,1 |
| Multivit. | Vitamine | 83,94 | 275,00 | 30,7 |
| Teufelskr. | Extrakt | 100,00 | 250,00 | 39,7 |
| B-Komplex | Vitamine | 126,2 | 420,00 | 30,0 |
| Sexoforte | Vitamine/Extrakte | 208,32 | 460,00 | 45,1 |
| Kerathym | Vitamine/Extrakte | 151,4 | 315,00 | 47,9 |

Aus dieser Auswertung ergibt sich unzweifelhaft, daß in bekannten Weichkapseln maximal 55 % Feststoffe, bezogen auf das Gesamtfüllgewicht, verkapselt werden können. Der Rest sind Öle und Emulgatoren. Demgegenüber kann bei den erfindungsgemäß hergestellten Kapseln der Hilfsstoffanteil drastisch reduziert werden, wodurch die Kapsel kleiner und billiger wird. Auf Emulgatoren (wie Lecithin) kann ebenfalls verzichtet werden.

**Patentansprüche**
**Patentansprüche für folgende Vertragsstaaten : AT, BE, CH, DE, DK, FR, GB, IT, LI, LU, NL, SE**

1. Weichgelatinekapsel, enthaltend einen in einem flüssigen Träger suspendierten oder gelösten phytopharmazeutischen Wirkstoff in Form eines mittels Wasser und/oder Alkohol extrahierten Pflanzenextrakts, **dadurch gekennzeichnet,** daß der Pflanzenextrakt ausschließlicher Wirkstoff und der flüssige Träger ein Lösungsmittel für den Pflanzenextrakt aus der Gruppe der polykondensierten Alkohole,

8

EP 0 464 274 B1

Polyole oder deren Monomethyl- bzw. Monoethylether ist.

**2.** Weichgelatinekapsel nach Anspruch 1, **dadurch gekennzeichnet,** daß dem Träger-Lösungsmittel biologische komplexbildende Lösungsvermittler der Phosphatidgruppe beigefügt sind.

**3.** Weichgelatinekapsel nach Anspruch 1 oder 2, **dadu rch gekennzeichnet,** daß das Lösungsmittel Polyethylenglykol ist.

**4.** Weichgelatinekapsel nach Anspruch 1, **dadurch gekennzeichnet,** daß das Lösungsmittel Polyglycerin ist.

**Patentansprüche für folgende Vertragsstaaten : ES, GR**

**1.** Verfahren zur Herstellung einer Weichgelatinekapsel unter Verwendung eines in einem flüssigen Träger suspendierten oder gelösten phytopharmazeutischen Wirkstoffs in Form eines mittels Wasser und/oder Alkohol extrahierten Pflanzenextrakts, **dadurch gekennzeichnet,** daß der Pflanzenextrakt als ausschließlicher Wirkstoff und als flüssiger Träger ein Lösungsmittel für den Pflanzenextrakt aus der Gruppe der polykondensierten Alkohole, Polyole oder deren Monomethyl- bzw. Monoethylether verwendet wird.

**2.** Verfahren zur Herstellung einer Weichgelatinekapsel nach Anspruch 1, **dadurch gekennzeichnet,** daß dem Träger-Lösungsmittel biologische komplexbildende Lösungsvermittler der Phosphatidgruppe beigefügt werden.

**3.** Verfahren zur Herstellung einer Weichgelatinekapsel nach Anspruch 1 oder 2, **dadurch gekennzeichnet,** daß als Lösungsmittel Polyethylenglykol verwendet wird.

**4.** Verfahren zur Herstellung einer Weichgelatinekapsel nach Anspruch 1, **dadurch gekennzeichnet,** daß als Lösungsmittel Polyglycerin verwendet wird.

**Claims**
**Claims for the following Contracting States : AT, BE, CH, DE, DK, FR, GB, IT, LI, LU, NL, SE**

**1.** A soft gelatine capsule, containing a phytopharmaceutical active substance suspended or dissolved in a liquid carrier, said active substance being in the form of a vegetable extract extracted by means of water or alcohol, characterized in that the vegetable extract is the exclusive active substance and the liquid carrier is a solvent for the vegetable extract of the group polycondensated alcohols, polyols or their monomethylethers or monoethylethers.

**2.** A soft gelatine capsule as claimed in claim 1, characterized in that biological complex forming solubilizers from the phosphatide group are added to the carrier solvent.

**3.** A soft gelatine capsule as claimed in claim 1 or 2, characterized in that the solvent is polyethylene glycol.

**4.** A soft gelatine capsule as claimed in claim 1, characterized in that the solvent is polyglycerol.

**Claims for the following Contracting States : ES, GR**

**1.** Method for fabricating a soft gelatine capsule using a phytopharmaceutical active substance suspended or dissolved in a liquid carrier, said active substance being in the form of a vegetable extract extracted by means of water or alcohol, characterized in that the vegetable extract is used as the exclusive active substance and a solvent for the vegetable extract of the group polycondensated alcohols, polyols or their monomethylethers or monoethylethers is used as the liquid carrier.

**2.** A method for fabricating a soft gelatine capsule as claimed in claim 1, characterized in that biological complex forming solubilizers from the phosphatide group are added to the carrier solvent.

9

3. A method for fabricating a soft gelatine capsule as claimed in claim 1 or 2, characterized in that polyethylene glycol is used as the solvent.

4. A method for fabricating a soft gelatine capsule as claimed in claim 1, characterized in that polyglycerol is used as the solvent.

**Revendications**
**Revendications pour les Etats contractants suivants : AT, BE, CH, DE, DK, FR, GB, IT, LI, LU, NL, SE**

1. Capsule en gélatine molle, contenant un principe actif phytopharmaceutique, en suspension ou dissous dans un excipient liquide, se présentant sous forme d'un extrait végétal extrait à l'eau et/ou à l'alcool, caractérisé en ce que l'extrait végétal est un principe actif exclusif et que l'excipient liquide est un solvant de l'extrait végétal, choisi parmi l'ensemble comprenant les alcools polycondensés, les polyols ou leur éther monométhylique ou monoéthylique.

2. Capsule en gélatine molle selon la revendication 1, caractérisée en ce qu'on a ajouté au solvant servant d'excipient des tiers-solvants complexants biologiques du groupe des phosphatides.

3. Capsule en gélatine molle selon la revendication 1 ou 2, caractérisée en ce que le solvant est le polyéthylèneglycol.

4. Capsule en gélatine molle selon la revendication 1, caractérisée en ce que le solvant est le polyglycérol.

**Revendications pour les Etats contractants suivants : ES, GR**

1. Procédé pour préparer une capsule en gélatine molle, par utilisation d'un principe actif phytopharmaceutique en suspension ou dissous dans un excipient liquide, se présentant sous la forme d'un extrait végétal extrait à l'eau et/ou à l'alcool, caractérisé en ce que l'extrait végétal est utilisé sous forme d'un principe actif exclusif et que l'on utilise comme excipient liquide un solvant de l'extrait végétal choisi parmi l'ensemble comprenant les alcools polycondensés, les polyols ou leur éther monométhylique ou monoéthylique.

2. Procédé pour préparer une capsule en gélatine molle selon la revendication 1, caractérisé en ce qu'on ajoute au solvant servant d'excipient des tiers-solvants complexants biologiques du groupe des phosphatides.

3. Procédé pour préparer une capsule en gélatine molle selon la revendication 1 ou 2, caractérisé en ce qu'on utilise comme solvant du polyéthylèneglycol.

4. Procédé pour préparer une capsule en gélatine molle selon la revendication 1, caractérisé en ce qu'on utilise comme solvant du polyglycérol.